# EUROPÄISCHE PATENTSCHRIFT

(11) **EP 4 094 715 B1**
(45) Veröffentlichungstag und Bekanntmachung des Hinweises auf die Patenterteilung: **04.10.2023**
(21) Anmeldenummer: 22173873.5
(22) Anmeldetag: 17.05.2022
(51) Int. Cl.: A61B 90/70, A47L 15/00

(54) **REINIGUNGS- UND/ODER DESINFEKTIONSGERÄT**
CLEANING AND/OR DISINFECTION MACHINE
APPAREIL DE NETTOYAGE ET/OU DE DÉSINFECTION

(30) Priorität: 27.05.2021 DE 102021113643
(43) Veröffentlichungstag der Anmeldung: 30.11.2022
(73) Patentinhaber: Olympus Winter & Ibe GmbH, 22045 Hamburg (DE)
(72) Erfinder: OEBBEKE, Dirk, 32547 Bad Oeynhausen (DE)
(74) Vertreter: Seemann & Partner Patentanwälte mbB

(56) Entgegenhaltungen:
- EP-B1- 0 432 281
- EP-B1- 3 409 824
- DE-A1-102017 129 860
- JP-A- 2002 253 466

## Beschreibung

Die Erfindung betrifft ein Reinigungs- und/oder Desinfektionsgerät, insbesondere Spülmaschine, mit einem einen Spülraum bereitstellenden Spülbehälter, der zur Beschickung mit zu reinigendem Spülgut eine Beschickungsöffnung aufweist, sowie mit einer Spülraumtür, mittels welcher die Beschickungsöffnung fluiddicht verschließbar ist, wobei die Spülraumtür eine translatorisch in Höhenrichtung des Spülbehälters verfahrbar ausgebildete Hubtür ist, wobei ein Tropfensammler aus einer in eine beschickungsöffnungsseitig über den Spülbehälter hinaus vorstehende Gebrauchsstellung und umgekehrt überführbar ist.

Ferner betrifft die Erfindung eine Verwendung eines Reinigungs- und/oder Desinfektionsgeräts.

Reinigungs- und/oder Desinfektionsgeräte der eingangs genannten Art sind aus dem Stand der Technik an sich gut bekannt, weshalb eines gesonderten druckschriftlichen Nachweises an dieser Stelle nicht bedarf. Es sei deshalb auch nur beispielhaft auf EP 2 604 296 A1 verwiesen.

Vorbekannte Reinigungs- und/oder Desinfektionsgeräte - kurz auch RDG genannt - verfügen typischerweise über einen Spülbehälter, der seinerseits einen Spülraum zur Aufnahme von zu reinigendem und/oder zu desinfizierendem Spülgut bereitstellt. Zwecks Beschickung des Spülraums mit zu reinigendem und/oder zu desinfizierendem Spülgut verfügt der Spülbehälter über eine Beschickungsöffnung. Diese ist mittels einer als Hubtür ausgebildeten Spülraumtür fluiddicht verschließbar.

Die Hubtür des aus der vorgenannten EP 2 604 296 A1 vorbekannten Reinigungs- und/oder Desinfektionsgeräts ist in Höhenrichtung des Spülraums translatorisch verfahrbar und kann hinsichtlich der Beschickungsöffnung aus einer Verschlussstellung in eine Offenstellung und umgekehrt verbracht werden. Dabei erfolgt ein Verfahren der Hubtür im bestimmungsgemäßen Verwendungsfall motorisch, zu welchem Zweck eine entsprechend ausgebildete Antriebseinrichtung vorgesehen ist.

Von grundlegender Problematik bei Reinigungs- und/oder Desinfektionsgeräten der gattungsgemäßen Art ist, dass bei in Offenstellung befindlicher Hubtür die Gefahr besteht, dass von der Hubtür selbst und/oder von aus dem Spülbehälter herausgefahrenen Spülgutträgern Restflüssigkeiten abtropfen können, was am Aufstellungsort des Reinigungs- und/oder Desinfektionsgeräts zu einem feuchten und verschmutzten Bodenbereich vor dem Reinigungs- und/oder Desinfektionsgerät führt. Dies ist nicht nur aus hygienischen Gründen problematisch, sondern stellt auch für einen Verwender ein Sicherheitsrisiko dar, da feuchte Bodenbereiche direkt vor dem Reinigungs- und/oder Desinfektionsgerät eine Rutschgefahr für Verwender darstellen. Bei Reinigungs- und/oder Desinfektionsgeräten mit verschwenkbar gelagerten Spülraumtüren tritt diese Problematik nicht auf, da eine in Offenstellung verschwenkte Spülraumtür einen Tropfschutz automatisch bereitstellt. Bei in Höhenrichtung vertikal verfahrbaren Hubtüren ist dieser automatische Tropfschutz aber nicht gegeben, weshalb insoweit Verbesserungsbedarf besteht.

In DE 10 2017 129 860 A1 ist ein Reinigungsgerät mit einer Hubtür beschrieben. Hierbei weist das Reinigungsgerät ein Türsystem mit einer Hubtür auf, wobei die Hubtür in einer Türebene durch eine translatorische Öffnungsbewegung zum Freigeben einer Öffnungszone bewegt wird.

Weiterhin beschreibt EP 3 409 824 B1 eine Waschvorrichtung mit einer Schiebetür mit einer Vorrichtung zum Sammeln und Entleeren des von der Tür tropfenden Wassers.

Des Weiteren ist in JP 2002 253 466 A eine Geschirrspülmaschine beschrieben, bei der das Abtropfen von Waschwasser nach dem Waschen verhindert wird.

Außerdem ist in EP 0 432 281 B1 eine Geschirrspülmaschine allgemein beschrieben.

Es ist ausgehend vom vorbeschriebenen Stand der Technik eine der Erfindung zugrundeliegende Aufgabe, ein Reinigungs- und/oder Desinfektionsgerät der gattungsgemäßen Art bereitzustellen, das hinsichtlich eines Tropfenschutzes konstruktiv weiterentwickelt ist, wobei es insbesondere darauf ankommt, den am Aufstellungsort des Reinigungs- und/oder Desinfektionsgeräts unmittelbar vor dem Reinigungs- und/oder Desinfektionsgerät befindlichen Bodenbereich von heruntertropfenden Restflüssigkeiten und/oder sonstigen Verunreinigungen weitestgehend frei zu halten.

Zur Lösung dieser Aufgabe wird mit der Erfindung ein Reinigungs- und/oder Desinfektionsgerät, insbesondere Spülmaschine, mit einem einen Spülraum bereitstellenden Spülbehälter, der zur Beschickung mit zu reinigendem Spülgut eine Beschickungsöffnung aufweist, sowie mit einer Spülraumtür, mittels welcher die Beschickungsöffnung fluiddicht verschließbar ist, wobei die Spülraumtür eine translatorisch in Höhenrichtung des Spülbehälters verfahrbar ausgebildete Hubtür ist, wobei ein Tropfensammler aus einer in eine beschickungsöffnungsseitig über den Spülbehälter hinaus vorstehende Gebrauchsstellung und umgekehrt überführbar ist, vorgeschlagen, das sich auszeichnet, dass der Tropfensammler in Höhenrichtung unterhalb des Spülbehälters angeordnet und translatorisch in einer quer zu Höhenrichtung des Spülbehälters verlaufenden Richtung verfahrbar ausgebildet ist, wobei der Tropfensammler aus einer in Überdeckung mit dem Spülbehälter stehenden Nicht-Gebrauchsstellung in die Gebrauchsstellung und umgekehrt überführbar ist.

Das Reinigungs- und/oder Desinfektionsgerät nach der Erfindung verfügt über einen Tropfensammler. Dieser dient dazu, bei in Offenstellung befindlicher Hubtür von der Hubtür und/oder von aus dem Spülbehälter heraus verfahrenen Spülgutträgern abtropfende Flüssigkeiten und/oder Verunreinigungen auffangen zu können. Damit ist in vorteilhafter Weise sichergestellt, dass der Bodenbereich in unmittelbarer Nähe vor dem Reinigungs- und/oder Desinfektionsgerät vor heruntertropfenden Restflüssigkeiten und/oder Verunreinigungen geschützt ist, dieser Bereich also frei von heruntertropfenden Restflüssigkeiten und/oder Verunreinigungen bleibt. Dies erlaubt im Unterschied zum Stand der Technik einen sehr viel hygienischeren Betrieb und vermindert zudem das Verletzungsrisiko, das infolge einer ansonsten bestehenden Rutschgefahr gegeben ist.

Das Reinigungs- und/oder Desinfektionsgerät ist insbesondere zur Aufbereitung von chirurgischen Instrumenten, insbesondere Endoskopen oder dergleichen vorgesehen, wobei insbesondere der Spülraum zur Aufnahme von einem oder mehreren, vorzugsweise in Aufnahmekörben, angeordneten chirurgischen Instrumenten ausgebildet ist. Um die Kanäle der chirurgischen Instrumente mit einem Spülkreislauf des Reinigungs- und/oder Desinfektionsgeräts, insbesondere Aufbereitungsgerät, zu verbinden, weisen die Aufnahmekörbe für die chirurgischen Instrumente, in dem die zu reinigenden Endoskope jeweils angeordnet sind, Adapter für die Kanäle der chirurgischen Instrumente, insbesondere Endoskope etc., auf. Dadurch werden beim Einbringen eines Aufnahmekorbs mit einem zu reinigenden chirurgischen Instrument, insbesondere Endoskop, in den Spülraum der Aufbereitungseinrichtung bzw. des Reinigungs- und/oder Desinfektionsgeräts die Kanäle des chirurgischen Instruments mit Spülkanälen des Spülkreislaufs oder der Adapter mit einem Gegenstück des Spülkreislaufs verbunden. Zur Reinigung sowie zur Desinfektion der chirurgischen Instrumente werden die Kanäle der chirurgischen Instrumente, insbesondere Endoskope mit einem Aufbereitungsfluid bzw. von Spülgut-, Reinigungs- und/oder Desinfektionsflüssigkeiten durchspült.

Im Ergebnis ist so ein in der Handhabung verbessertes Reinigungs- und/oder Desinfektionsgerät gegeben, denn es ist insbesondere nicht mehr erforderlich, den Bodenbereich vor dem Reinigungs- und/oder Desinfektionsgerät verwenderseitig manuell durch entsprechende Reinigungstätigkeiten sauber zu halten. Mittels des erfindungsgemäß vorgesehenen Tropfensammlers werden etwaige von der Hubtür oder von aus dem Spülraum herausgefahrenen Spülgutträgern, zum Beispiel Aufnahmekörbe für mit darin angeordneten chirurgischen Instrumenten, abtropfende Restflüssigkeiten und/oder Verunreinigungen abgefangen und zentral gesammelt und/oder abgeführt.

Der erfindungsgemäße Tropfensammler ist in Höhenrichtung unterhalb des Spülbehälters angeordnet und translatorisch in einer quer zur Höhenrichtung des Spülbehälters verlaufenden Richtung verfahrbar ausgebildet. Dabei ist der Tropfensammler aus einer in Überdeckung mit dem Spülbehälter stehenden Nicht-Gebrauchsstellung in eine beschickungsöffnungsseitig über den Spülbehälter hinaus vorstehende Gebrauchsstellung und umgekehrt überführbar.

Der Tropfensammler ist außerhalb des vom Spülbehälter bereitgestellten Spülraums angeordnet, nämlich unterhalb des Spülbehälters. Im Bedarfsfall kann der Tropfensammler aus einer Nicht-Gebrauchsstellung in eine Gebrauchsstellung und umgekehrt überführt werden, wobei der Tropfensammler linear verfahrbar ist, und zwar in einer quer zur Höhenrichtung des Spülbehälters verlaufenden Richtung.

In der Nicht-Gebrauchsstellung befindet sich der Tropfensammler in Überdeckung mit dem Spülbehälter, das heißt der Tropfensammler ist in dieser Stellung unmittelbar unterhalb des Spülbehälters angeordnet und steht nicht über diesen hinaus vor. Eine völlig freie Zugänglichkeit zum Spülbehälter ist so gewährleistet.

Zur Überführung des Tropfensammlers in die Gebrauchsstellung ist dieser linear zu verfahren, und zwar in Richtung der beschickungsöffnungsseitigen Wandung des Spülbehälters. Sobald der Tropfensammler seine Endposition in Gebrauchsstellung erreicht hat, steht der Tropfensammler schubladengleich über den Spülbehälter hinaus vor, und zwar beschickungsöffnungsseitig. Damit liegt der Boden am Aufstellungsort, der sich direkt vor dem Reinigungs- und/oder Desinfektionsgerät befindet, im Schatten des Tropfensammlers, so dass abtropfende Restflüssigkeiten und/oder Verunreinigungen nicht auf den Boden, sondern auf den Tropfensammler fallen. Durch den Tropfensammler ist mithin der direkt darunterliegende Boden vor abtropfenden Restflüssigkeiten und/oder herunterfallenden Verschmutzungen und/oder dergleichen Verunreinigungen geschützt.

Gemäß einem weiteren Merkmal der Erfindung ist vorgesehen, dass der Tropfensammler ein plattenförmiges Element aufweist. Besonders bevorzugt ist es, den Tropfensammler als Platte auszubilden. Es ist so ein insgesamt in Höhenrichtung flacher Aufbau gegeben, was eine platzsparende Anordnung des Tropfensammlers unterhalb des Spülbehälters ermöglicht.

Gemäß einem weiteren Merkmal der Erfindung ist vorgesehen, dass die dem Spülbehälter zugewandte Großseite des Flächenelements ein Gefälle aufweist. Hierdurch ist sichergestellt, dass etwaige vom Tropfensammler aufgefangene Tropfen und/oder Verunreinigungen dem Gefälle folgend automatisch, das heißt unter Einwirkung der Gewichtskraft abtransportiert werden können. Dabei ist es bevorzugt, dass sich an dem Tropfensammler zumindest in Nicht-Gebrauchsstellung eine Einrichtung befindet, mittels welcher vom Tropfensammler in Entsprechung des Gefälles abgegebene Restfeuchtigkeit und/oder Verunreinigungen abgefördert und/oder einer vom Reinigungs- und/oder Desinfektionsautomat ohnehin bereitgestellten Abwasserpumpe zugeleitet werden können.

Gemäß einem weiteren Merkmal der Erfindung ist vorgesehen, dass der Tropfensammler nach Art einer Wanne ausgebildet ist, wobei das Flächenelement spülraumseitig einen Umrandungssteg aufweist.

Diese Ausgestaltung stellt in vorteilhafter Weise sicher, dass der Tropfensammler eine gewisse Aufnahmekapazität bereitstellt, so dass ein randseitiges Übertreten von Feuchtigkeit oder Schmutz wirkungsvoll verhindert ist.

Gemäß einem weiteren Merkmal der Erfindung ist vorgesehen, dass der Tropfensammler mittels Kugelrollenauszüge verfahrbar gelagert ist. Diese Ausgestaltung stellt eine sichere und leichtgängige Verfahrbewegung des Tropfensammlers sicher und sorgt für eine lange Lebensdauer. Zudem ist eine präzise Führung des Tropfensammlers sichergestellt.

Gemäß einem weiteren Merkmal der Erfindung ist vorgesehen, dass der Tropfensammler manuell verfahrbar ist. Er kann mithin verwenderseitig wahlweise in Benutzung genommen und aus der Gebrauchsstellung in die Nicht-Gebrauchsstellung bzw. umgekehrt überführt werden. Dabei ist der Tropfensammler verwenderseitig zu ergreifen und nach Art einer Schublade wahlweise translatorisch zu verfahren, das heißt einzufahren bzw. auszufahren.

Gemäß einem weiteren Merkmal der Erfindung ist vorgesehen, dass der Tropfensammler motorisch verfahrbar ist. Gemäß dieser Ausführungsform ist ein elektromotorischer Antrieb für den Tropfensammler vorgesehen, so dass ein motorbewegtes Ausfahren bzw. Einfahren des Tropfensammlers gestattet ist. Ein solches Verfahren kann entweder automatisch in Abhängigkeit der Stellung der Hubtür oder unabhängig hiervon erfolgen, das heißt wahlweise durch den Verwender.

Gemäß einem weiteren Merkmal der Erfindung ist vorgesehen, dass der Tropfensammler mit einer verwenderseitig separat bedienbaren Motoreinrichtung in Wirkverbindung steht. Gemäß dieser Ausführungsform kann ein Verfahren des Tropfensammlers unabhängig von der Stellung der Hubtür erfolgen. Zu diesem Zweck ist eine für den Tropfensammler separat vorgesehene Motoreinrichtung ausgebildet. Diese Motoreinrichtung kann verwenderseitig wahlweise bedient werden, zu welchem Zweck eine entsprechende Bedieneinheit vorgesehen ist.

Gemäß einem weiteren Merkmal der Erfindung ist vorgesehen, dass die Hubtür motorisch verfahrbar ist, zu welchem Zweck eine Motoreinheit vorgesehen ist, die in Wirkverbindung mit der Hubtür steht. Gemäß dieser Ausgestaltung findet keine manuelle Bewegung der Hubtür aus der Verschlussstellung in die Offenstellung und umgekehrt statt, stattdessen ist ein elektromotorischer Antrieb vorgesehen. Der Verwender kann wahlweise quasi auf Knopfdruck über die elektromotorische Einheit eine Bewegung der Hubtür einleiten.

Es ist gemäß einem besonders bevorzugten Merkmal der Erfindung vorgesehen, dass der Tropfensammler in Wirkverbindung mit der Motoreinheit der Hubtür steht. Demgemäß wird die für die Hubtür ohnehin vorgesehene Motoreinheit auch dazu benutzt, den Tropfensammler verfahren zu können. Da die Hubtür einerseits und der Tropfensammler andererseits ein und dieselbe Motoreinheit nutzen bzw. mit dieser in Wirkverbindung stehen, ist eine Zwangskopplung von Hubtür und Tropfensammler vorgesehen. Diese Zwangskopplung bewirkt, dass bei einem Verfahren der Hubtür auch der Tropfensammler automatisch mit verfährt. Ein manuelles Einschreiten eines Verwenders ist insofern in vorteilhafter Weise nicht erforderlich.

Dabei ist es bevorzugterweise vorgesehen, dass bei einem Überführen der Hubtür aus der Verschlussstellung in die Offenstellung der Tropfensammler automatisch ausfährt und in schon vorbeschriebener Weise einen Tropfschutz bildet. Wird die Hubtür hingegen aus der Offenstellung in die Verschlussstellung zurückverfahren, so verfährt auch der Tropfensammler zurück in seine Nicht-Gebrauchsstellung, in welcher er direkt unterhalb des Spülbehälters in Parkposition angeordnet ist.

Gemäß einem weiteren Merkmal der Erfindung ist vorgesehen, dass die Motoreinheit der Hubtür einen Elektromotor und eine damit zusammenwirkende Antriebswelle aufweist, wobei die Antriebswelle mit einem einen Mitnehmer aufweisenden Kraftübertragungsmittel zusammenwirkt.

Die für die Hubtür vorgesehene Motoreinheit verfügt über einen Elektromotor und eine Antriebswelle. Im bestimmungsgemäßen Verwendungsfall wird die Antriebswelle mittels des Elektromotors in eine Verdrehbewegung versetzt. Die Antriebswelle wirkt wiederum ihrerseits mit einem Kraftübertragungsmittel zusammen, bei dem es sich beispielsweise um einen Riemen, vorzugsweise einen Keilriemen oder einen Zahnriemen, oder eine Kette handeln kann. Dieses Kraftübertragungsmittel verfügt über einen Mitnehmer, der wiederum mit dem Tropfensammler zusammenwirkt. Auf diese Weise ist eine kraftübertragende Kopplung zwischen dem Elektromotor der Motoreinheit einerseits und dem Tropfensammler andererseits realisiert, so dass bei einem bestimmungsgemäßen Betrieb des Elektromotors der Motoreinheit der Tropfensammler verfährt. Dabei fährt der Tropfensammler je nach Drehrichtung des Elektromotors entweder aus der Gebrauchsstellung in die Nicht-Gebrauchsstellung oder umgekehrt.

Gemäß einem weiteren Merkmal der Erfindung ist vorgesehen, dass der Mitnehmer formschlüssig in eine vom Tropfensammler bereitgestellte Aufnahme eingreift. Auf diese Weise ist die Kraftkopplung zwischen Mitnehmer einerseits und Tropfensammler andererseits gegeben. Dabei ist diese Wirkverbindung in vorteilhafter Weise formschlüssig ausgebildet, was insbesondere eine leichte Montage im Reparaturfall ermöglicht.

Gemäß einem weiteren Merkmal der Erfindung ist vorgesehen, dass die Aufnahme ein Schlitz ist, der eine die Abmessung des Mitnehmers in Längsrichtung übersteigende Längserstreckung aufweist. Demgemäß ist der Schlitz in Längsrichtung größer ausgebildet als der Mitnehmer. Es entsteht so eine Schlitz-Leerstrecke, was bedeutet, dass der Mitnehmer zunächst über eine gewisse Strecke innerhalb des Schlitzes verfährt, bevor es zu einem kraftübertragenden Anliegen des Mitnehmers am jeweiligen Schlitzende kommt. Es ist so eine Art Getriebestufe realisiert, womit sichergestellt ist, dass zunächst die Hubtür über eine gewisse Strecke in Höhenrichtung verfährt, bevor der Tropfensammler seinerseits angetrieben wird. Ungewollte Kollisionen zwischen Tropfensammler einerseits und Hubtür andererseits sind somit vermieden. Zudem gestattet es diese Ausgestaltung, je nach Aufstellungsort und Verwenderwunsch über die Größe des Mitnehmers die Getriebeübersetzung zwischen Mitnehmer und Schlitz sowie die Größe der Leerstrecke einstellen zu können. Individuellen Bedienungsanforderungen kann so in vorteilhafter Weise Rechnung getragen werden.

Gemäß einem weiteren Merkmal der Erfindung ist vorgesehen, dass zwischen dem Elektromotor und dem Kraftübertragungsmittel eine Getriebeanordnung vorgesehen ist. Durch eine solche Getriebeanordnung kann ferner das Übersetzungsverhältnis zwischen einer Verfahrbewegung der Hubtür und einer Verfahrbewegung des Tropfensammlers eingestellt und gegebenenfalls auch individuell nachträglich verändert werden.

Im Ergebnis der erfindungsgemäßen Ausgestaltung ergibt sich insgesamt eine kostengünstige Realisierung. Dies insbesondere dann, wenn für einen motorischen Antrieb des Tropfensammlers der ohnehin für die Hubtür vorgesehene motorische Antrieb genutzt wird.

Der Tropfensammler ist unterhalb des Spülbehälters angeordnet, das heißt nicht innerhalb des vom Spülbehälter bereitgestellten Spülraums, womit eine unerwünschte Verschattung innerhalb des Spülraums vermieden ist.

Von der Hubtür und/oder aus dem Spülraum herausgefahrenen Spülgutträgern herabtropfende Flüssigkeit kann in einfacher Weise durch Leitbleche oder gegebenenfalls durch eine leichte Neigung des Tropfensammlers abgeführt werden, gegebenenfalls in einen Bereich des Reinigungs- und/oder Desinfektionsgeräts, der ohnehin mit einer Abfördereinrichtung in strömungstechnischer Verbindung steht. Vorzugsweise sind die Spülgutträger als Aufnahmekörbe für chirurgische Instrumente, insbesondere Endoskope, ausgebildet, in denen jeweils mindestens ein chirurgisches Instrument für die Aufbereitung angeordnet wird oder anordbar ist.

Die erfindungsgemäße Ausgestaltung ist darüber hinaus grundsätzlich unabhängig von der Verfahrrichtung der Hubtür, so dass der erfindungsgemäße Tropfensammler sowohl in Kombination mit solchen Hubtüren eingesetzt werden kann, die zur Überführung in die Offenstellung in Höhenrichtung nach oben verfahren, als auch mit solchen Hubtüren, die zur Überführung in die Offenstellung in Höhenrichtung nach unten verfahren.

Zudem ist von Vorteil, dass der erfindungsgemäße Tropfensammler unterhalb des Spülbehälters angeordnet ist, was es bei einem als Tunnelgerät ausgebildeten Gerät ermöglicht, den Tropfensammler entweder zu der einen oder zu der anderen Seite verfahren zu können, das heißt auf die sogenannte "saubere" Seite oder die sogenannte "schmutzige" Seite.

Ferner wird die Aufgabe gelöst durch eine Verwendung eines voranstehend beschriebenen Reinigungs- und/oder Desinfektionsgeräts, insbesondere Spülmaschine, zur Aufbereitung von wenigstens einem chirurgischen Instrument, insbesondere Endoskop. Zur Vermeidung von Wiederholungen wird auf die obigen Ausführungen ausdrücklich verwiesen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen. Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen

Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird.

Weitere Merkmale und Vorteile der Erfindung ergeben sich aus der nachfolgenden Beschreibung anhand der Figuren. Dabei zeigen
- Fig. 1: in schematischer Frontansicht ein erfindungsgemäßes Reinigungs- und/oder Desinfektionsgerät;
- Fig. 2: in schematischer Seitenansicht das Reinigungs- und/oder Desinfektionsgerät nach Fig. 1;
- Fig. 3: in schematischer Ansicht von unten das Reinigungs- und/oder Desinfektionsgerät nach Fig. 1;
- Fig. 4: in schematisch perspektivischer Darstellung den erfindungsgemäßen Tropfensammler samt Motoreinheit;
- Fig. 5: in schematisch perspektivischer Darstellung aus einem anderen Blickwinkel den Tropfensammler nach Fig. 4;
- Fig. 6: in schematischer Frontansicht das erfindungsgemäße Reinigungs- und/oder Desinfektionsgerät mit teilgeöffneter Hubtür;
- Fig. 7: in schematischer Seitenansicht das Reinigungs- und/oder Desinfektionsgerät nach Fig. 6;
- Fig. 8: in schematischer Detailansicht den Ausschnitt X nach Fig. 7;
- Fig. 9: in schematischer Ansicht von unten das Reinigungs- und/oder Desinfektionsgerät nach Fig. 6;
- Fig. 10: in einer Frontansicht das erfindungsgemäße Reinigungs- und/oder Desinfektionsgerät mit voll geöffneter Hubtür;
- Fig. 11: in schematischer Seitenansicht das Reinigungs- und/oder Desinfektionsgerät nach Fig. 10;
- Fig. 12: in einer Ausschnittdarstellung den Ausschnitt X nach Fig. 11;
- Fig. 13: in schematischer Ansicht von unten das Reinigungs- und/oder Desinfektionsgerät nach Fig. 10;
- Fig. 14: in schematischer Frontansicht das erfindungsgemäße Reinigungs- und/oder Desinfektionsgerät mit zum Teil geschlossener Hubtür;
- Fig. 15: in schematischer Seitenansicht das Reinigungs- und/oder Desinfektionsgerät nach Fig. 14;
- Fig. 16: in einer Ausschnittdarstellung den Ausschnitt X nach Fig. 15 und
- Fig. 17: in einer schematischen Ansicht von unten das Reinigungs- und/oder Desinfektionsgerät nach Fig. 14.

In den Zeichnungen sind jeweils gleiche oder gleichartige Elemente und/oder Teile mit denselben Bezugsziffern versehen, so dass von einer erneuten Vorstellung jeweils abgesehen wird.

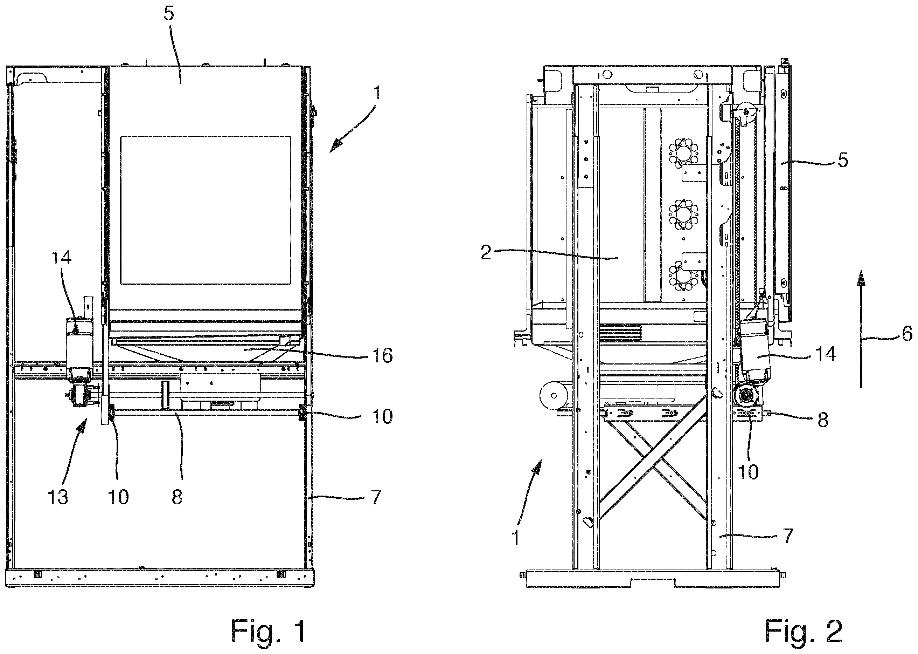

Die Zeichungen Fig. 1 bis Fig. 17 zeigen in unterschiedlichen Ansichten ein erfindungsgemäßes Reinigungs- und/oder Desinfektionsgerät 1, kurz RDG genannt. Das Reinigungs- und/oder Desinfektionsgerät 1 ist vorzugsweise zur Aufbereitung von chirurgischen Instrumenten, insbesondere Endoskopen ausgebildet.

Das Reinigungs- und/oder Desinfektionsgerät 1 verfügt über einen Spülbehälter 2, der einen Spülraum 3 zur Aufnahme von zu reinigendem und/oder zu desinfizierendem Spülgut, insbesondere chirurgische Instrumente wie zum Beispiel Endoskope, bereitstellt. Der Spülraum 3 ist über ein Beschickungsöffnung 4 zugänglich, die mittels einer in Höhenrichtung 6 translatorisch verfahrbaren Hubtür 5 fluiddicht verschließbar ist. Zur tragenden Abstützung des Spülbehälters 2 und der daran verfahrbar angeordneten Hubtür 5 dient ein Gestell 7.

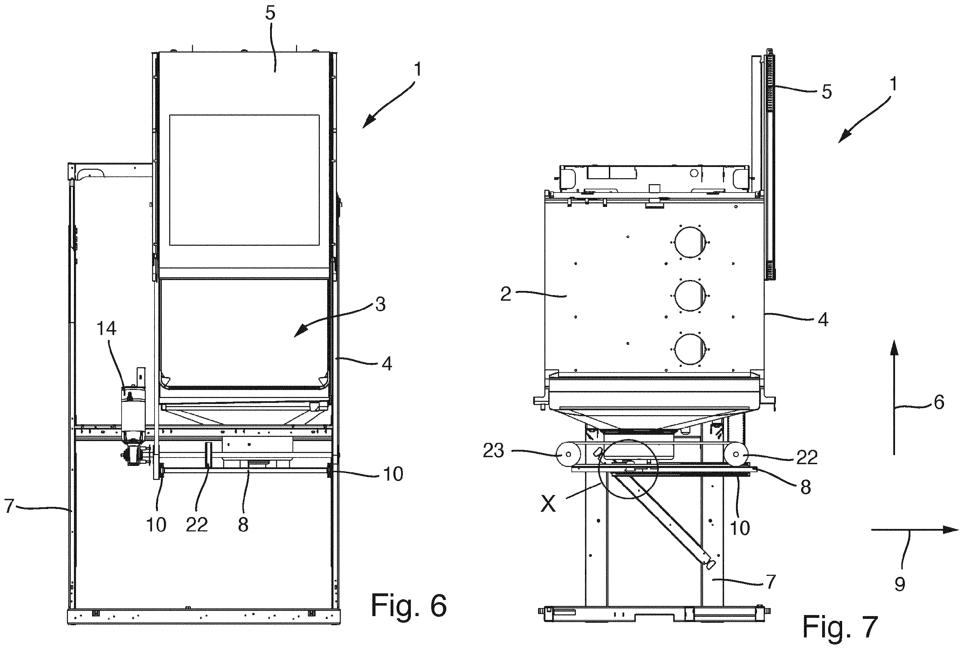

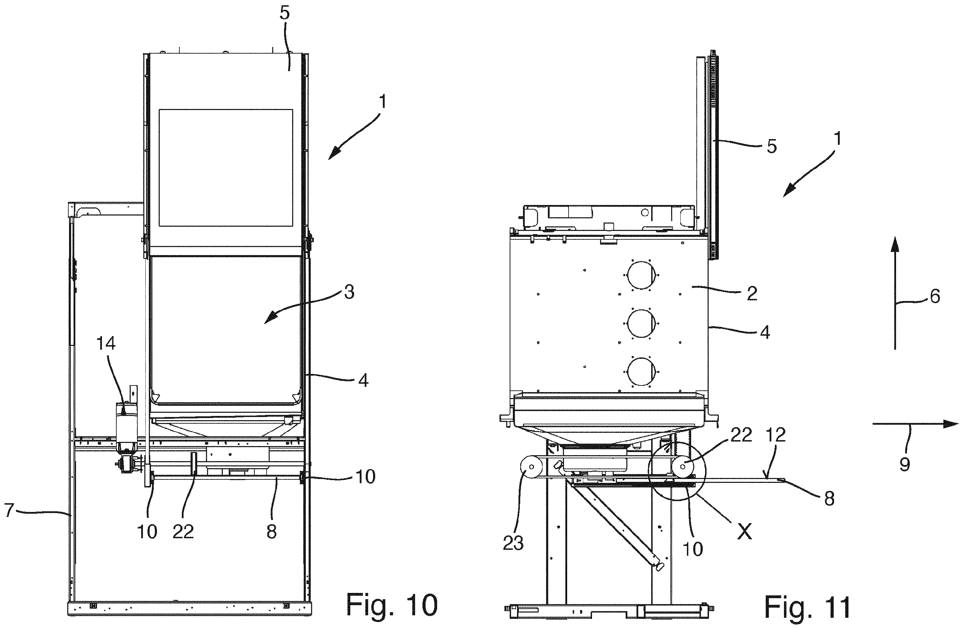

Die Zeichnungen Fig. 1 bis Fig. 3 zeigen das erfindungsgemäße Reinigungs- und/oder Desinfektionsgerät 1 mit sich in Verschlussstellung befindlicher Hubtür 5. Die Figuren 6 bis 9 zeigen das erfindungsgemäße Reinigungs- und/oder Desinfektionsgerät 1 mit einer teilgeöffneten Hubtür 5, das heißt einer Hubtür 5, die teilweise in Höhenrichtung 6 nach oben verfahren ist. Die Figuren 10 bis 13 zeigen schließlich das erfindungsgemäße Reinigungs- und/oder Desinfektionsgerät 1 mit einer in Offenstellung befindlichen Hubtür 5, das heißt einer Hubtür 5, die in Höhenrichtung 6 vollständig in ihre Öffnungsstellung verfahren ist.

Das erfindungsgemäße Reinigungs- und/oder Desinfektionsgerät 1 verfügt über einen Tropfensammler 8. Dieser ist in Höhenrichtung 6 unterhalb des Spülraums 3 angeordnet und nach Art eines plattenförmigen Flächenelements 11 ausgebildet.

Der Tropfensammler 8 ist mittels zweier Kugelrollauszüge 10 translatorisch verfahrbar gelagert und in einer quer zur Höhenrichtung 6 verlaufenden Querrichtung 9 beweglich. Er kann aus einer in den Figuren 1 bis 3 gezeigten Nicht-Gebrauchsstellung in eine in den Figuren 10 bis 13 gezeigte Gebrauchsstellung und umgekehrt verfahren werden, und zwar nach Art einer Schublade. Zur Aufnahme des sich an den Spülbehälter 2 anschließenden Sammeltopfes 16 ist der Tropfensammler 8 mit einem Ausschnitt 17 ausgerüstet.

Die dem Spülbehälter 2 zugewandte Großseite 12 des Flächenelements 11 ist mit einem Gefälle ausgebildet, so dass vom Tropfensammler 8 etwaig aufgefangene Tropfen und/oder Verunreinigungen in Richtung einer zentralen Aufnahme abströmen können.

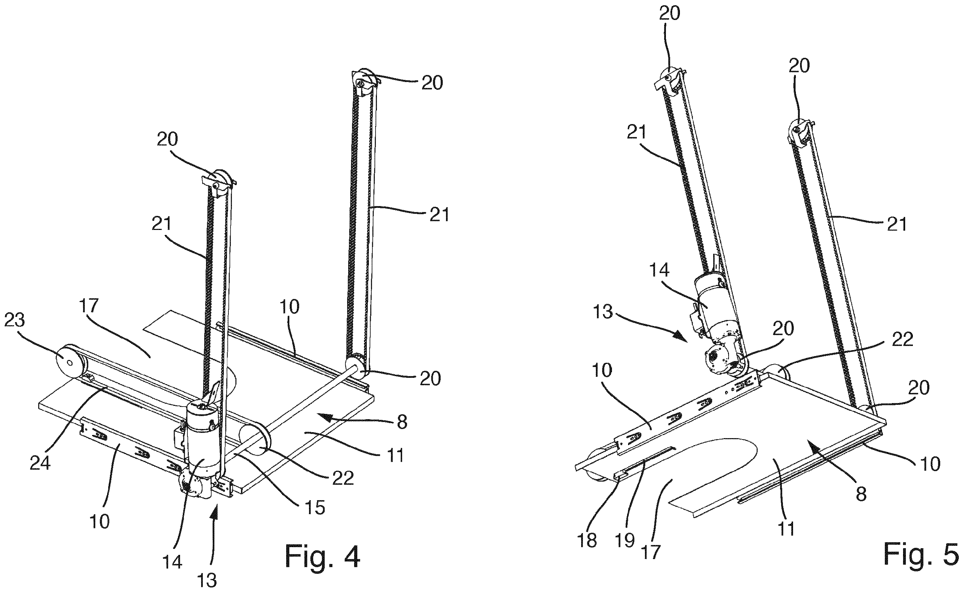

Die Hubtür 5 ist motorisch verfahrbar ausgebildet. Zu diesem Zweck ist eine Motoreinheit 13 vorgesehen, die über einen Elektromotor 14 und eine Antriebswelle 15 verfügt, wie dies insbesondere die Figuren 4 und 5 erkennen lassen. Die Antriebswelle 15 ist im gezeigten Ausführungsbeispiel mit Zahnrädern 20 bestückt, über die jeweils ein Zahnriemen 21 geführt ist, der anderendseitig über gestellseitige Zahnräder 20 geführt ist. Im bestimmungsgemäßen Verwendungsfall wird bei einer Inbetriebnahme des Elektromotors 14 die Antriebswelle 15 verdreht, was zu einem Antrieb der Zahnriemen 21 führt, was wiederum eine Bewegung der Hubtür 5 in Höhenrichtung 6 zur Folge hat.

Der linear verfahrbare Tropensammler 8 steht mit der Motoreinheit 13 in Wirkverbindung. Zu diesem Zweck sind zwei Räder 22 und 23 vorgesehen, wobei das Rad 22 verdrehfest auf der Antriebswelle 15 angeordnet ist. Über die beiden Räder 22 und 23 ist ein Kraftübertragungsmittel in Form eines Keilriemens 24 geführt. Dieser ist mit einem Mitnehmer 18 ausgerüstet, der in einer als Schlitz 19 ausgebildete Ausnehmung des Tropfensammlers 8 eingreift, wie dies insbesondere die Darstellung nach Fig. 5 erkennen lässt. Auf diese Weise ist eine Zwangskopplung zwischen Hubtürbewegung einerseits und Tropfensammlerbewegung andererseits gegeben. Diese Zwangskopplung führt dazu, dass bei einer Überführung der Hubtür 5 in die Offenstellung der Tropfensammler 8 mit Bezug auf die Zeichnungsebene beispielsweise nach Fig. 7 nach rechts verfährt. Bei einer Rücküberführung der Hubtür 5 zurück in die Verschlussstellung erfolgt indes eine Verfahrbewegung des Tropfensammlers 8 zurück in die Nicht-Gebrauchsstellung, das heißt beispielsweise mit Bezug auf die Zeichnungsebene nach Fig. 5 nach links.

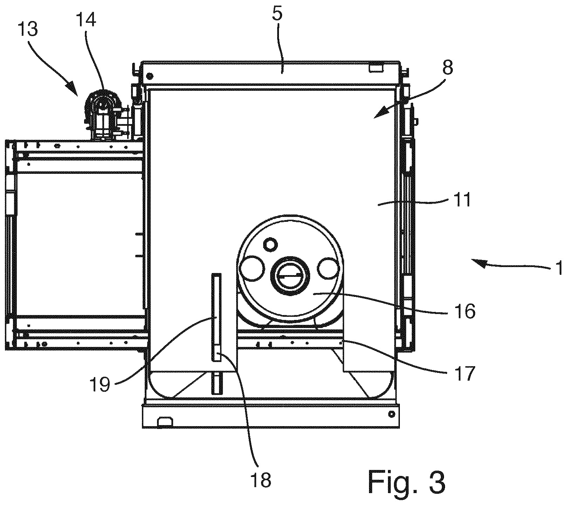

Der Schlitz 19 weist eine Längserstreckung auf, die die Längserstreckung des Mitnehmers 18 übersteigt. Aufgrund dessen ist eine Leerstrecke gegeben, die sicherstellt, dass eine Bewegung der Hubtür 5 nicht zu einer sofortigen Bewegung auch des Tropfensammlers 8 führt. Vielmehr hat die Hubtür 5 erst über eine gewisse Wegstrecke zu verfahren, bevor dann auch der Tropfensammler 8 verfährt. Fig. 3 lässt in diesem Zusammenhang die Stellung des Mitnehmers 18 bei vollständig geschlossener Hubtür 5 erkennen. Danach liegt der Mitnehmer 18 an der mit Bezug auf die Zeichnungsebene nach Fig. 3 unteren Randkante des Schlitzes 19 an.

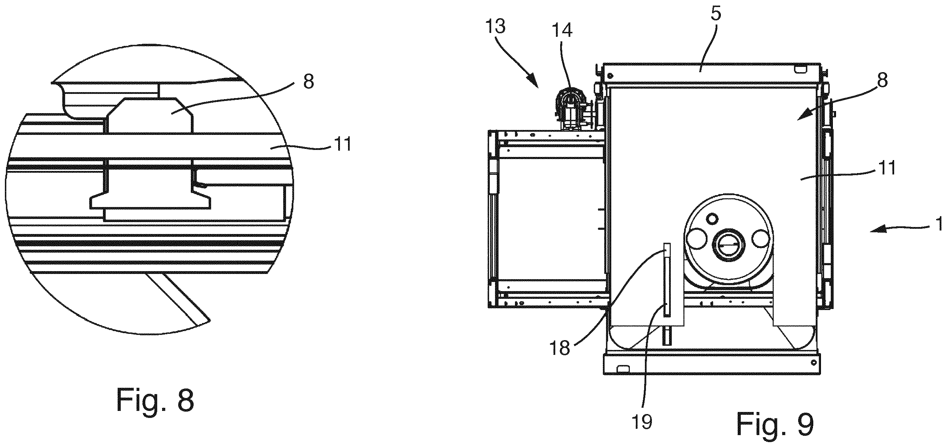

Sobald die Hubtür 5 in ihre Offenstellung bewegt wird, wandert der Mitnehmer 18 innerhalb des Schlitzes 19, bis er die in Fig. 9 gezeigte Stellung erreicht. In dieser Stellung ist die Hubtür 5 bereits etwas geöffnet, der Tropfensammler 8 aber nach wie vor nicht verfahren. Der Mitnehmer 18 liegt nun an der mit Bezug auf die Zeichnungsebene nach Fig. 9 oberen Randkante des Schlitzes 19 an.

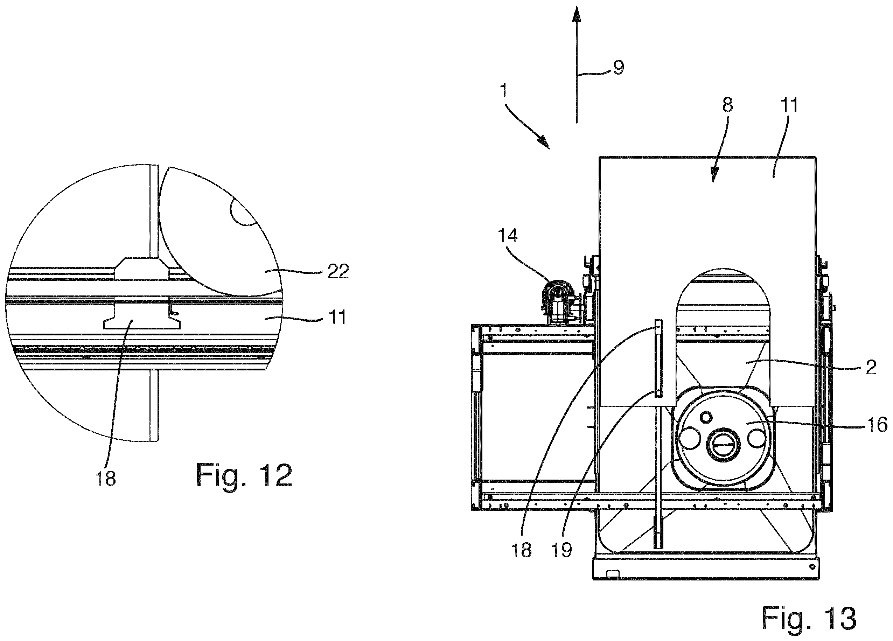

Wird die Hubtür 5 nun in Entsprechung der Figuren 10 und 11 weiter bis in die vollständig geöffnete Offenstellung verfahren, so nimmt der Mitnehmer 18 nunmehr den Tropfensammler 8 mit, wodurch dieser in seine ausgefahrene Gebrauchsstellung verbracht wird, wie dies insbesondere Fig. 13 erkennen lässt.

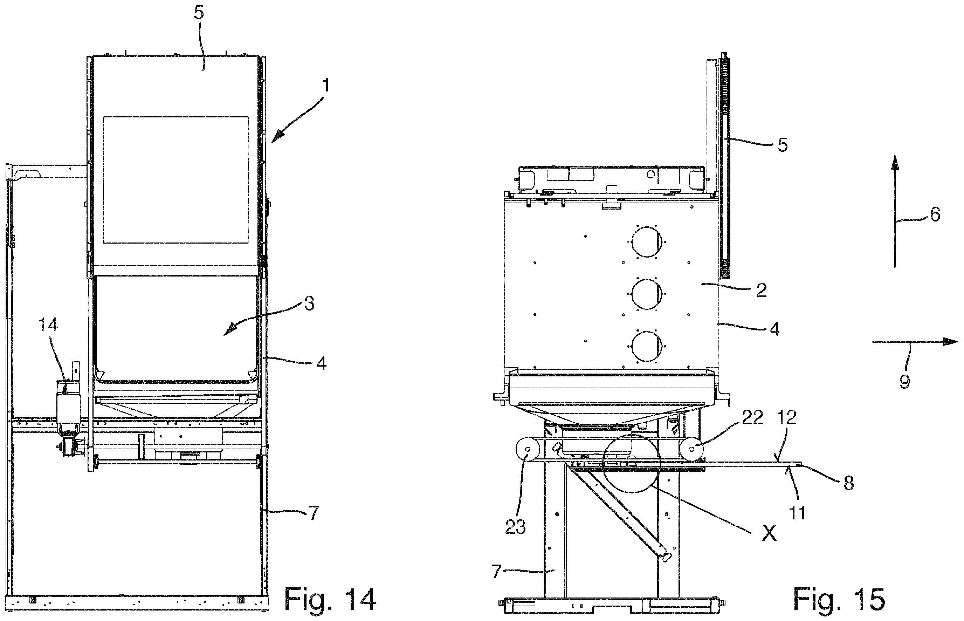

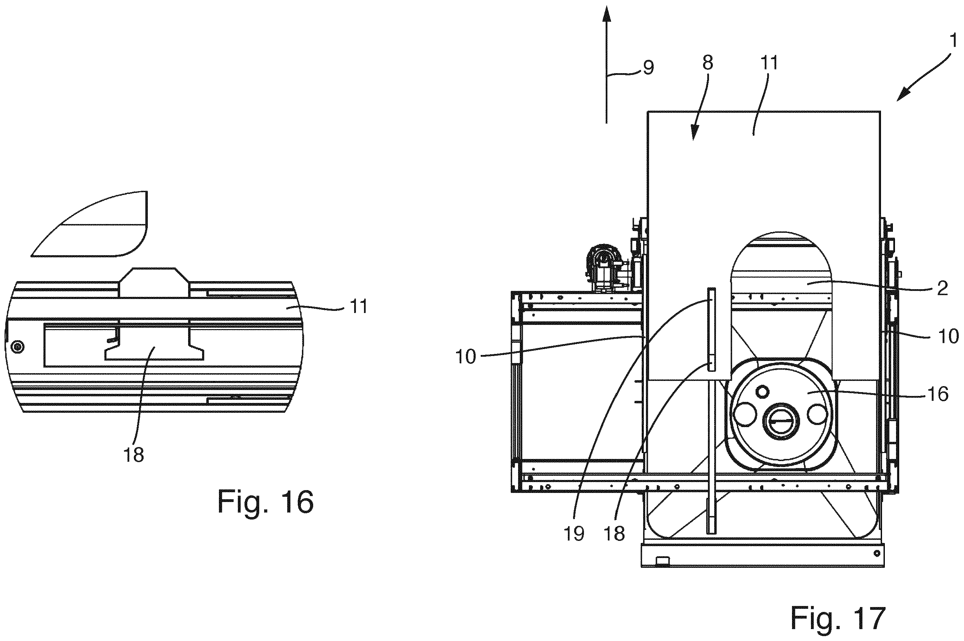

Wird die Hubtür 5 alsdann wieder verschlossen, das heißt zurück in die Verschlussstellung verbracht, so bleibt der Tropfensammler 8 zunächst in seiner ausgefahrenen Gebrauchsstellung, wie dies die Figuren 14 und 15 erkennen lassen. In dieser Gebrauchsstellung verbleibt der Tropfensammler 8 so lange, bis der Mitnehmer 18 innerhalb des Schlitzes 19 wieder soweit verfahren ist, dass er mit Bezug auf die Zeichnungsebene nach Fig. 17 an der unteren Randkante des Schlitzes 19 angekommen ist.

Sobald dies geschehen ist, führt ein weiteres Verfahren der Hubtür 5 dazu, dass der Tropfensammler 8 mitgenommen und zurück in seine Nicht-Gebrauchsstellung gemäß der Figuren 1 und 2 verfahren wird.

Sobald sich der Tropfensammler 8 in seiner Gebrauchsstellung beispielsweise gemäß Fig. 11 befindet, dient er als Tropfschutz für den unterhalb des Tropfensammlers 8 befindlichen Bodenbereich. Etwaige von der Hubtür 5 oder von aus dem Spülbehälter unter Umständen herausgefahrenen Spülgutträgern abtropfende Restflüssigkeit und/oder Verschmutzungen werden durch den Tropfensammler 8 abgefangen und gelangen nicht ungewollt in den unterhalb des Tropfensammlers 8 befindlichen Bodenbereich.

Erfindungsgemäße Ausführungsformen können durch einzelne Merkmale oder eine Kombination mehrerer Merkmale erfüllt sein.

### Bezugszeichen

- 1: Reinigungs- und/oder Desinfektionsgerät
- 2: Spülbehälter
- 3: Spülraum
- 4: Beschickungsöffnung
- 5: Hubtür
- 6: Höhenrichtung
- 7: Gestell
- 8: Tropfensammler
- 9: Querrichtung
- 10: Kugelrollauszug
- 11: Flächenelement
- 12: Großseite
- 13: Motoreinheit
- 14: Elektromotor
- 15: Antriebswelle
- 16: Sammeltopf
- 17: Ausschnitt
- 18: Mitnehmer
- 19: Schlitz
- 20: Zahnrad
- 21: Zahnriemen
- 22: Rad
- 23: Rad
- 24: Keilriemen

## Patentansprüche

1. Reinigungs- und/oder Desinfektionsgerät, insbesondere Spülmaschine, mit einem einen Spülraum (3) bereitstellenden Spülbehälter (2), der zur Beschickung mit zu reinigendem Spülgut eine Beschickungsöffnung (4) aufweist, sowie mit einer Spülraumtür, mittels welcher die Beschickungsöffnung (4) fluiddicht verschließbar ist, wobei die Spülraumtür eine translatorisch in Höhenrichtung (6) des Spülbehälters (2) verfahrbar ausgebildete Hubtür (5) ist, wobei ein Tropfensammler (8) aus einer in eine beschickungsöffnungsseitig über den Spülbehälter (2) hinaus vorstehende Gebrauchsstellung und umgekehrt überführbar ist, **dadurch gekennzeichnet, dass** der Tropfensammler (8) in Höhenrichtung (6) unterhalb des Spülbehälters (2) angeordnet und translatorisch in einer quer zur Höhenrichtung (6) des Spülbehälters (2) verlaufenden Richtung (9) verfahrbar ausgebildet ist, wobei der Tropfensammler (8) aus einer in Überdeckung mit dem Spülbehälter (2) stehenden Nicht-Gebrauchsstellung in die Gebrauchsstellung und umgekehrt überführbar ist.

2. Gerät nach Anspruch 1, **dadurch gekennzeichnet, dass** der Tropfensammler (8) ein plattenförmiges Flächenelement (11) aufweist.

3. Gerät nach Anspruch 2, **dadurch gekennzeichnet, dass** die dem Spülbehälter (2) zugewandte Großseite (12) des Flächenelements (11) ein Gefälle aufweist.

4. Gerät nach Anspruch 2 oder 3, **dadurch gekennzeichnet, dass** der Tropfensammler (8) nach Art einer Wanne ausgebildet ist, wobei das Flächenelement (11) spülraumseitig einen Umrandungssteg aufweist.

5. Gerät nach einem der vorhergehenden Ansprüche, **dadurch gekennzeichnet, dass** der Tropfensammler (8) mittels Kugelrollauszügen (10) verfahrbar gelagert ist.

6. Gerät nach einem der vorhergehenden Ansprüche, **dadurch gekennzeichnet, dass** der Tropfensammler (8) manuell verfahrbar ist.

7. Gerät nach einem der vorhergehenden Ansprüche, **dadurch gekennzeichnet, dass** der Tropfensammler (8) motorisch verfahrbar ist.

8. Gerät nach Anspruch 7, **dadurch gekennzeichnet, dass** der Tropfensammler (8) mit einer verwenderseitig separat bedienbaren Motoreinrichtung in Wirkverbindung steht.

9. Gerät nach einem der vorhergehenden Ansprüche, **dadurch gekennzeichnet, dass** die Hubtür (5) motorisch verfahrbar ist, zu welchem Zweck eine Motoreinheit (13) vorgesehen ist, die in Wirkverbindung mit der Hubtür (5) steht.

10. Gerät nach Anspruch 9, **dadurch gekennzeichnet, dass** der Tropfensammler (8) in Wirkverbindung mit der Motoreinheit (13) der Hubtür (5) steht.

11. Gerät nach Anspruch 9 oder 10, **dadurch gekennzeichnet, dass** die Motoreinheit (13) der Hubtür (5) einen Elektromotor (14) und eine damit zusammenwirkende Antriebswelle (15) aufweist, wobei die Antriebswelle (15) mit einem einen Mitnehmer (18) aufweisenden Kraftübertragungsmittel zusammenwirkt.

12. Gerät nach Anspruch 11, **dadurch gekennzeichnet, dass** der Mitnehmer (18) formschlüssig in eine vom Tropfensammler (8) bereitgestellte Aufnahme eingreift, wobei insbesondere die Aufnahme ein Schlitz (19) ist, der eine die Abmessung des Mitnehmers (18) in Längsrichtung übersteigende Längserstreckung aufweist.

13. Gerät nach Anspruch 11 oder 12, **dadurch gekennzeichnet, dass** zwischen Elektromotor (14) und Kraftübertragungsmittel eine Getriebeanordnung vorgesehen ist.

14. Gerät nach einem der vorhergehenden Ansprüche 11 bis 13, **dadurch gekennzeichnet, dass** das Kraftübertragungsmittel ein Riemen (24) oder eine Kette ist.

15. Verwendung eines Reinigungs- und/oder Desinfektionsgeräts, insbesondere Spülmaschine, nach einem der Ansprüche 1 bis 14 zur Aufbereitung von wenigstens einem chirurgischen Instrument, insbesondere Endoskop.

## Claims

1. A cleaning and/or disinfecting device, in particular a rinsing machine, with a rinsing container (2) providing a rinsing chamber (3), which container has a loading opening (4) for loading with washware to be cleaned, as well as with a rinsing chamber door, by means of which the loading opening (4) can be closed in a fluid-tight manner, wherein the rinsing chamber door is a lift door (5) configured to be displaceable translationally in the height direction (6) of the rinsing container (2), wherein a drip collector (8) can be transitioned from a use position protruding past the rinsing container (2) on the loading opening side and vice versa, **characterized in that** the drip collector (8) which is arranged below the rinsing container (2) in the height direction (6) and is configured to be displaced translationally in a direction (9) running transversely to the height direction (6) of the rinsing container (2), wherein the drip collector (8) can be transitioned from a non-use position overlapping with the rinsing container (2) to the use position and vice versa.

2. The device according to claim 1, **characterized in that** the drip collector (8) has a plate-shaped flat element (11).

3. The device according to claim 2, **characterized in that** the large side (12), facing the rinsing container (2), of the flat element (11) has a slope.

4. The device according to claim 2 or 3, **characterized in that** the drip collector (8) is configured as a type of trough, wherein the flat element (11) has a border strip on the rinsing chamber side.

5. The device according to one of the preceding claims, **characterized in that** the drip collector (8) is mounted to be displaceable by means of ball roller pull-outs (10).

6. The device according to one of the preceding claims, **characterized in that** the drip collector (8) can be displaced manually.

7. The device according to one of the preceding claims, **characterized in that** the drip collector (8) can be displaced by a motor.

8. The device according to claim 7, **characterized in that** the drip collector (8) is in operative connection with a motor device that can be operated separately by a user.

9. The device according to one of the preceding claims, **characterized in that** the lift door (5) can be displaced by a motor, for which purpose a motor unit (13) is provided that is in operative connection with the lift door (5).

10. The device according to claim 9, **characterized in that** the drip collector (8) is in operative connection with the motor unit (13) of the lift door (5).

11. The device according to claim 9 or 10, **characterized in that** the motor unit (13) of the lift door (5) has an electric motor (14) and a drive shaft (15) interacting with it, wherein the drive shaft (15) interacts with a force transmission means having a driver (18).

12. The device according to claim 11, **characterized in that** the driver (18) engages in a form-fitting manner in a receiver provided by the drip collector (8), wherein in particular the receiver is a slot (19) that has a longitudinal extent exceeding the dimension of the driver (14) in the longitudinal direction.

13. The device according to claim 11 or 12, **characterized in that** a gear arrangement is provided between the electric motor (14) and force transmission means.

14. The device according to one of the preceding claims 11 to 13, **characterized in that** the force transmission means is a belt (24) or a chain.

15. A use of a cleaning and/or disinfection device, in particular a rinsing machine, according to one of claims 1 to 14 for the reprocessing of at least one surgical instrument, in particular an endoscope.

## Revendications

1. Appareil de nettoyage et/ou de désinfection, en particulier lave-vaisselle, avec une cuve de lavage (2) pourvue d'une chambre de lavage (3), qui présente une ouverture de chargement (4) pour le chargement de la vaisselle à nettoyer, ainsi qu'avec une porte de chambre de lavage, au moyen de laquelle l'ouverture de chargement (4) peut être fermée de manière étanche aux fluides, la porte de chambre de lavage étant une porte relevable (5) conçue de manière à pouvoir se déplacer en translation dans le sens de la hauteur (6) de la cuve de lavage (2), un collecteur de gouttes (8) étant apte à être déplacé depuis une position d'utilisation vers une position d'utilisation dépassant de la cuve de lavage (2) du côté de l'ouverture de chargement, et inversement, **caractérisé en ce que** le collecteur de gouttes (8) est disposé dans le sens de la hauteur (6) en dessous de la cuve de lavage (2) et est conçu de manière à être apte à être déplacé en translation dans une direction (9) s'étendant transversalement au sens de la hauteur (6) de la cuve de lavage (2), le collecteur de gouttes (8) étant apte à être transféré d'une position de non-utilisation se trouvant en recouvrement avec la cuve de lavage (2) à la position d'utilisation et inversement.

2. Appareil selon la revendication 1, **caractérisé en ce que** le collecteur de gouttes (8) présente un élément de surface (11) en forme de plaque.

3. Appareil selon la revendication 2, **caractérisé en ce que** le grand côté (12) de l'élément de surface (11) tourné vers la cuve de lavage (2) présente une pente.

4. Appareil selon la revendication 2 ou la revendication 3, **caractérisé en ce que** le collecteur de gouttes (8) est conçu à la manière d'une cuve, l'élément de surface (11) présentant une nervure de bordure du côté de la chambre de lavage.

5. Appareil selon l'une des revendications précédentes, **caractérisé en ce que** le collecteur de gouttes (8) est monté de manière à pouvoir être déplacé au moyen de glissières à billes (10).

6. Appareil selon l'une des revendications précédentes, **caractérisé en ce que** le collecteur de gouttes (8) peut être déplacé manuellement.

7. Appareil selon l'une des revendications précédentes, **caractérisé en ce que** le collecteur de gouttes (8) peut être déplacé par un moteur.

8. Appareil selon la revendication 7, **caractérisé en ce que** le collecteur de gouttes (8) est en liaison active avec un dispositif à moteur pouvant être commandé séparément par l'utilisateur.

9. Appareil selon l'une quelconque des revendications précédentes, **caractérisé en ce que** la porte relevable (5) peut être déplacée par un moteur, une unité motrice (13) étant prévue à cet effet, laquelle est en liaison fonctionnelle avec la porte relevable (5).

10. Appareil selon la revendication 9, **caractérisé en ce que** le collecteur de gouttes (8) est en liaison active avec l'unité motrice (13) de la porte relevable (5).

11. Appareil selon la revendication 9 ou la revendication 10, **caractérisé en ce que** l'unité motrice (13) de la porte relevable (5) comporte un moteur électrique (14) et un arbre d'entraînement (15) coopérant avec celui-ci, l'arbre d'entraînement (15) coopérant avec un moyen de transmission de puissance comportant un entraîneur (18).

12. Appareil selon la revendication 11, **caractérisé en ce que** l'entraîneur (18) est en engagement par complémentarité de forme dans un logement prévu sur le collecteur de gouttes (8), dans lequel, en particulier le logement est une fente (19) qui a une extension longitudinale dépassant la dimension de l'entraîneur (18) dans la direction longitudinale.

13. Appareil selon la revendication 11 ou 12, **caractérisé en ce qu'**un agencement de transmission est prévu entre le moteur électrique (14) et le moyen de transmission de puissance.

14. Appareil selon l'une quelconque des revendications 11 à 13 précédentes, **caractérisé en ce que** le moyen de transmission de puissance est une courroie (24) ou une chaîne.

15. Utilisation d'un appareil de nettoyage et/ou de désinfection, en particulier lave-vaisselle, selon l'une des revendications 1 à 14, pour le traitement d'au moins un instrument chirurgical, en particulier un endoscope.
